# EUROPEAN PATENT SPECIFICATION

(11) **EP 3 072 957 B1**
(45) Date of publication and mention of the grant of the patent: **10.07.2019**
(21) Application number: 16161739.4
(22) Date of filing: 22.03.2016
(51) Int. Cl.: C12N 5/00, C12P 21/00

(54) **METHODS FOR CONTROLLING PROTEIN GLYCOSYLATION**
VERFAHREN ZUR STEUERUNG DER PROTEINGLYCOSYLIERUNG
PROCÉDÉS PERMETTANT DE RÉGULER LA GLYCOSYLATION DE PROTÉINES

(30) Priority: 23.03.2015 US 201562136850 P
(43) Date of publication of application: 28.09.2016
(73) Proprietor: Lonza Ltd, 3930 Visp (CH); Glaxo Group Limited, Brentford, Middlesex TW8 9GS (GB)
(72) Inventor: BERI, Rajesh G., Portsmouth, NH 03801 (US); HADLEY, Brian, Portsmouth, NH 03801 (US); HEIMBACH, Jim, Portsmouth, NH 03801 (US); MAXWELL, Allan, Portsmouth, NH 03801 (US); CANNING, Valerie, Portsmouth, NH 03801 (US); GERBER, Robert George, Telford, PA 18969 (US)
(74) Representative: Eder, Michael

(56) References cited:
- WO-A1-2015/026846
- WO-A2-2012/149197
- P. HOSSLER ET AL: "Optimal and consistent protein glycosylation in mammalian cell culture", GLYCOBIOLOGY, vol. 19, no. 9, 3 June 2009 (2009-06-03), pages 936-949, XP055112498, ISSN: 0959-6658, DOI: 10.1093/glycob/cwp079
- YUK INN H Y ET AL: "Changes in the overall extent of protein glycosylation by Chinese hamster ovary cells over the course of batch culture", BIOTECHNOLOGY AND APPLIED BIOCHEMISTRY, ACADEMIC PRESS, US, vol. 36, no. 2, 1 October 2002 (2002-10-01), pages 133-140, XP009175566, ISSN: 0885-4513
- FENG LI ET AL: "Cell culture processes for monoclonal antibody production", MABS, vol. 2, no. 5, 1 September 2010 (2010-09-01), pages 466-479, XP055166177, ISSN: 1942-0862, DOI: 10.4161/mabs.2.5.12720
- Michel Chartrain ET AL: "Development and Production of Commercial Therapeutic Monoclonal An- tibodies in Mammalian Cell Expression Systems: An Overview of the Cur- rent Upstream Technologies", Current Pharmaceutical Biotechnology, 1 January 2008 (2008-01-01), pages 447-467, XP055288072, Retrieved from the Internet: URL:http://ic.ucsc.edu/~drsmith/metx270/ht ml/Chartrain and Chu.pdf.pdf
- RYAN BONIFACE ET AL: "Profiling of glycosylation gene expression in CHO fed-batch cultures in response to glycosylation-enhancing medium components", BMC PROCEEDINGS, BIOMED CENTRAL LTD, LONDON UK, vol. 7, no. Suppl 6, 4 December 2013 (2013-12-04), page P99, XP021170402, ISSN: 1753-6561, DOI: 10.1186/1753-6561-7-S6-P99
- DANNY CHEE FURNG WONG ET AL: "Profiling of N-glycosylation gene expression in CHO cell fed-batch cultures", BIOTECHNOLOGY AND BIOENGINEERING, vol. 107, no. 3, 15 October 2010 (2010-10-15), pages 516-528, XP055085544, ISSN: 0006-3592, DOI: 10.1002/bit.22828
- YUZHOU FAN ET AL: "Amino acid and glucose metabolism in fed-batch CHO cell culture affects antibody production and glycosylation", BIOTECHNOLOGY AND BIOENGINEERING., vol. 112, no. 3, 1 March 2015 (2015-03-01) , pages 521-535, XP055288000, US ISSN: 0006-3592, DOI: 10.1002/bit.25450

## Description

### BACKGROUND

### 1. Field

The present disclosure relates to processes for improving and/or ensuring batch-to-batch consistency of glycosylation in recombinant proteins.

### 2. Description of Related Art

Exogenous glycoproteins that may be obtained from eukaryotic cells include, for example, recombinant therapeutic glycoproteins (*e.g*., interleukin 2, erythropoietin, tissue plasminogen activator, antithrombin III, and antibodies). Usually, such glycoproteins are obtained from mammalian cells, and they may be useful for various conditions including, for example, haemophilia, diabetes, anemia, cancer, and autoimmune and inflammatory diseases.

For example, monoclonal antibodies (mAbs) play an important role in the treatment of cancer, and autoimmune and inflammatory diseases. Recombinant monoclonal and/or polyclonal antibodies (rmAb and rpAb, respectively) can be manufactured in and purified from a production bioreactor as a single preparation without separate handling, manufacturing, purification, or characterization of the individual members constituting the recombinant polyclonal protein. This production strategy, however, requires a robust procedure for verifying the identity and demonstrating consistent production of antibody molecules over time; the same is true for any recombinant glycoprotein.

The investigation, development, and production of therapeutic glycoproteins, and their official and/or clinical licensing, requires elaborate analysis with regard to *in-vivo* half life, biological safety, product definition, and batch consistency. The glycosylation of recombinant proteins, especially those destined for administration to human subjects, is of critical importance. Glycosylation profoundly affects biological activity, function, clearance from circulation, and crucially, antigenicity. The cells of nonhuman species do not glycosylate their proteins in the same way as human cells do, and in many cases the differences are profound. Consequently, the glycosylation of recombinant proteins constitutes one of the critical quality attributes that requires thorough analysis for optimal efficacy and safety. Methods to control protein glycosylation have been described in the art. For example, WO 2015/026846 A1 to Biogen Idee, Inc. discloses a cell culture medium comprising media supplements that are shown to control recombinant protein glycosylation and/or cell culture in a controlled or modulated (shifted) temperature to control recombinant protein glycosylation and/or cell culture with controlled or modulated seed density to control recombinant protein glycosylation.

Nevertheless, achieving consistent glycosylation of recombinant proteins produced in non-native cell types remains difficult. Despite apparent consistency of production procedures between batches, glycosylation of recombinant proteins can vary between batches, altering the behavior and efficacy of bio therapeutic drugs. Thus, improved control over batch-to-batch variations of glycosylation in glycoprotein products is needed.

The solution to this technical problem is provided by the embodiments characterized in the claims.

### BRIEF SUMMARY

The present disclosure provides, in one embodiment, methods of reducing the variability of glycosylation levels between batches of a recombinant protein having a target glycosylation range, and said target glycosylation range having a midpoint, the method comprising: (a) providing a cell suitable for expressing said recombinant protein; (b) determining upper and lower viable cell density (VCD) levels for a seed culture of a cell (N-1 VCD), determining upper and lower viable cell density levels for a production culture of said cell (N VCD), and determining the midpoint between said N-1 VCD upper and lower levels, and the midpoint between said N VCD upper and lower levels; wherein the upper and lower viable cell density levels are the range, i.e., values between and including the upper and lower viable cell density levels, of viable cell densities at which recombinant protein having a glycosylation level within the target glycosylation range is obtained; (c) culturing said cells to a viable cell density which is between 80% and 120% of the midpoint between said N-1 VCD upper and lower levels, thereby producing a constrained seed culture; and (d) inoculating a culture medium with said constrained seed culture to make a constrained production culture, culturing said constrained production culture to a viable cell density between said N VCD midpoint and lower levels, and then feeding said constrained production culture; wherein steps (c) and (d) yield a first batch of said recombinant protein from said cell, wherein said glycosylation level of said recombinant protein is within said target glycosylation range; (e) repeating steps (c) through (d) to yield at least one subsequent batch of said recombinant protein from said cell of step (a), wherein the glycosylation levels of said first and at least one subsequent batch are between about 75% to about 125% of the target glycosylation range midpoint, and/or the coefficient of variation of the glycosylation levels of said recombinant proteins of said first and at least one subsequent batch is 4.5% or less, wherein the coefficient of variation is the standard deviation (σ) divided by the mean value (µ)..

In some embodiments, the glycosylation levels of said first and at least one subsequent batch are between about 50% to about 150%, about 55% to about 145%, about 60% to about 140%, about 65% to about 135%, about 70% to about 130%, about 80% to about 120%, about 85% to about 115%, about 90% to about 110%, about 95% to about 105%, about 97.5% to about 102.5%, and preferably between about 75% to about 125%, of the target glycosylation range midpoint.

In some embodiments, the coefficient of variation is 4.5%, 4.4%, 4.3%, 4.2%, 4.1%, 4.0%, 3.9%, 3.8%, 3.7%, 3.6%, 3.5%, 3.4%, 3.2%, 3.1%, 3.0%, 2.9%, 2.8%, 2.7%, 2.6%, 2.5%, 2.4%, 2.3%, 2.2%, 2.1%, 2.0%, 1.9%, 1.8%, 1.7%, 1.6%, 1.5%, 1.4%, 1.3%, 1.2%, 1.1%, 1.0%, 0.9%, 0.8%, 0.7%, 0.6%, 0.5%, 0.4%, 0.3%, 0.2%, 0.1, or less.

In some embodiments, the cells of the seed culture (step (c)) may be cultured to a viable cell density which is between about 30% and about 170%, about 40% and about 160%, about 50% and about 150%, about 60% and about 140%, about 70% and about 130%, about 90% and about 110%, and preferably between about 80% and about 120%, of the midpoint between said N-1 VCD upper and lower levels.

In some embodiments, the ratio of the VCD of the cells of the seed culture of step (c) versus the midpoint between said N-1 VCD upper and lower levels is from about 0.25 to about 1.75, from about 0.30 to about 1.70, from about 0.40 to about 1.65, from about 0.45 to about 1.60, from about 0.50 to about 1.55, from about 0.55 to about 1.50, from about 0.60 to about 1.45, from about 0.65 to about 1.40, from about 0.70 to about 1.35, from about 0.75 to about 1.30, from about 0.80 to about 1.25, from about 0.80 to about 1.20, from about 0.85 to about 1.15, from about 0.90 to about 1.10, from about 0.95 to about 1.05, from about 0.80 to about 1.05, from about 0.85 to about 1.05, and preferably from about from about 0.80 to about 1.20.

In some embodiments, the cell is a eukaryotic cell. In some embodiments, the cell is a fungal cell, a plant cell, an insect cell, or a mammalian cell. In some embodiments, the cell is selected from the group consisting of CHO, CHOK1SV, NS0, SP2/0, PERC.6, myeloma, and hybridoma cells.

In some embodiments, the N-1 VCD is from about 10 x 10⁵ to about 75 x 10⁵ cells/mL and the N VCD is from about 10 x 10⁵ to about 50 x 10⁵ cells/mL.

In some embodiments, the N-1 VCD is from about 10 x 10⁵ to about 50 x 10⁵ cells/mL and said N VCD is from about 10 x 10⁵ to about 30 x 10⁵ cells/mL.

In some embodiments, the N-1 VCD is from about 10 x 10⁵ to about 25 x 10⁵ cells/mL and the N VCD is from about 10 x 10⁵ to about 20 x 10⁵ cells/mL.

In some embodiments, the N-1 VCD is from about 15 x 10⁵ to about 25 x 10⁵ cells/mL and the N VCD is from about 15 x 10⁵ to about 20 x 10⁵ cells/mL.

In some embodiments, the feeding of the constrained production culture is after about 6 to about 200, about 12 to about 150, about 18 to about 125, about 20 to about 120, about 22 to about 110, or about 24 to about 108 hours following said inoculating with the constrained seed culture.

In some embodiments, the glycosylation is selected from glycans G0F, G1F, G2F, and combinations thereof. In some embodiments, the glycosylation is selected from glycans G0, G1, G2, and combinations thereof.

In some embodiments, glycan G0F comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 30% to about 50% of said glycosylation.

In some embodiments, glycan G1F comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 25% to about 35% of said glycosylation.

In some embodiments, glycan G2F comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 5% to about 15% of said glycosylation.

In some embodiments, glycan G0 comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 30% to about 50% of said glycosylation.

In some embodiments, glycan G1 comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 25% to about 35% of said glycosylation.

In some embodiments, glycan G2 comprises from about 5% to about 95%, from about 10% to about 80%, from about 15% to about 75%, from about 20% to about 70%, from about 25% to about 65%, from about 30% to about 60%, from about 30% to about 55%, from about 30% to about 50%, from about 30% to about 45%, from about 30% to about 40%, from about 30% to about 35%, from about 5% to about 30%, from about 5% to about 25%, from about 5% to about 20%, from about 5% to about 15%, and from about 5% to about 10%, and preferably from about 5% to about 15% of said glycosylation.

In some embodiments, the target glycosylation range is a predetermined pharmaceutical product specification or a quality control criterion.

In some embodiments, the recombinant protein is a recombinant therapeutic protein. In some embodiments, the recombinant therapeutic protein is a recombinant therapeutic antibody. In some embodiments, the target glycosylation range is a glycosylation range of one or more glycans in a preparation of a recombinant therapeutic antibody.

In some embodiments, the recombinant protein is selected from the group consisting of CD proteins such as CD2, CD3, CD4, CD8, CD11, CD19, CD20, CD22, CD25, CD33, CD34, CD40, CD52; members of the ErbB receptor family such as the EGF receptor (EGFR, HER1, ErbBI), HER2 (ErbB2), HER3 (ErbB3) or HER4 (ErbB4) receptor; macrophage receptors such as CRIg; tumor necrosis factors such as TNFa or TRAIL/Apo-2; cell adhesion molecules such as LFA-1, Macl, p150,95, VLA-4, ICAM-1, VCAM and αvβ3 integrin including either a or β subunits thereof (*e.g*., anti-CD11a, anti-CD18 or anti-CD11b antibodies); growth factors and receptors such as EGF, FGFR (*e.g*., FGFR3) and VEGF; IgE; cytokines such as IL1; cytokine receptors such as IL2 receptor; blood group antigens; flk2/flt3 receptor; obesity (OB) receptor; mpl receptor; CTLA-4; protein C; neutropilins; ephrins and receptors; netrins and receptors; slit and receptors; chemokines and chemokine receptors such as CCL5, CCR4, CCR5; amyloid beta; complement factors, such as complement factor D; lipoproteins, such as oxidized LDL (oxLDL); lymphotoxins, such as lymphotoxin alpha (LTa). Other molecular targets include Tweak, B7RP-1, proprotein convertase subtilisin/kexin type 9 (PCSK9), sclerostin, c-kit, Tie-2, c-fms, and anti-M1.

In some embodiments, the recombinant protein is abatacept, abciximab, adalimumab, aflibercept, alefacept, alemtuzumab, basiliximab, belatacept, belimumab, bevacizumab, canakinumab, brentuximab vedotin, certolizumab, cetuximab, daclizumab, denileukin diftitox, denosumab, eculizumab, efalizumab, etanercept, gemtuzumab, golimumab, ibritumomab, infliximab, ipilimumab, muromonab, natalizumab, ofatumumab, omalizumab, palivizumab, panitumumab, ranibizumab, rilonacept, rituximab, tocilizumab, tositumomab, or trastuzumab.

### DETAILED DESCRIPTION

Before the subject disclosure is further described, it is to be understood that the disclosure is not limited to the particular embodiments of the disclosure described below, as variations of the particular embodiments may be made and still fall within the scope of the appended claims. It is also to be understood that the terminology employed is for the purpose of describing particular embodiments, and is not intended to be limiting. Instead, the scope of the present disclosure will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this disclosure belongs.

The present disclosure teaches the surprising finding that the batch-to-batch consistency of a recombinant polypeptide (*e.g*., an antibody) having a target glycosylation range can be improved by modulating the viable cell density (VCD) of a seed culture (N-1 VCD), by modulating the viable cell density of a production culture (N VCD), and/or by initiating feeding of the production culture in accord with said N VCD.

### Definitions

As used herein, "polypeptide" (or "amino acid sequence" or "protein") refers to an oligopeptide, peptide, polypeptide, or protein sequence, and fragments or portions thereof, and to naturally occurring or synthetic molecules. "Amino acid sequence" and like terms, such as "polypeptide" or "protein", are not meant to limit the indicated amino acid sequence to the complete, native amino acid sequence associated with the recited protein molecule.

The term "treatment" or "treating", as used herein, refers to administering a therapy in an amount, manner, and/or mode effective to improve a condition, symptom, or parameter associated with a disorder or condition or to prevent or reduce progression of a disorder or condition, to a degree detectable to one skilled in the art. An effective amount, manner, or mode can vary depending on the subject and may be tailored to the subject.

As used herein, the term "antibody" refers to a polypeptide that includes at least one immunoglobulin variable region (*e.g*., an amino acid sequence that provides an immunoglobulin variable domain or immunoglobulin variable domain sequence). For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (*e.g*., single chain antibodies, Fab, F(ab')₂, Fd, Fv, and dAb fragments) as well as complete antibodies (*e.g*., intact immunoglobulins of types IgA, IgG, IgE, IgD, IgM, as well as subtypes thereof). The light chains of the immunoglobulin can be of types kappa or lambda. In some embodiments, an antibody includes an Fc region. In some embodiments, an antibody is a therapeutic antibody.

As used herein, the term "Fc region" refers to a dimer of two "Fc polypeptides", each "Fc polypeptide" comprising the constant region of an antibody excluding the first constant region immunoglobulin domain. In some embodiments, an "Fc region" includes two Fc polypeptides linked by one or more disulfide bonds, chemical linkers, or peptide linkers. "Fc polypeptide" refers to the last two constant region immunoglobulin domains of IgA, IgD, and IgG, and the last three constant region immunoglobulin domains of IgE and IgM, and may also include part or all of the flexible hinge N-terminal to these domains. For IgG, "Fc polypeptide" comprises immunoglobulin domains Cgamma2 (Cγ2) and Cgamma3 (Cγ3) and the lower part of the hinge between CgammaI (CγI) and Cγ2. Although the boundaries of the Fc polypeptide may vary, the human IgG heavy chain Fc polypeptide is usually defined to comprise residues starting at T223 or C226 or P230, to its carboxyl-terminus, wherein the numbering is according to the EU index as in Kabat *et al.* (1991, NIH Publication 91-3242, National Technical Information Services, Springfield, VA). For IgA, Fc polypeptide comprises immunoglobulin domains Calpha2 (Cα2) and Calpha3 (Cα3) and the lower part of the hinge between CalphaI (CαI) and Cα2. An Fc region can be synthetic, recombinant, or generated from natural sources such as IVIG.

As used herein, a "glycan" is a sugar. Glycans can be monomers or polymers of sugar residues, but typically contain at least three sugars, and can be linear or branched. A glycan may include natural sugar residues (*e.g*., glucose, N-acetylglucosamine, N-acetyl neuraminic acid, galactose, mannose, fucose, hexose, arabinose, ribose, xylose, etc.) and/or modified sugars (*e.g*., 2'-fluororibose, 2'-deoxyribose, phosphomannose, 6'-sulfo N-acetylglucosamine, etc). The term "glycan" includes homo and heteropolymers of sugar residues. The term "glycan" also encompasses a glycan component of a glycoconjugate (*e.g*., of a glycoprotein, glycolipid, proteoglycan, etc.). The term also encompasses free glycans, including glycans that have been cleaved or otherwise released from a glycoconjugate.

As used herein, a "galactosylated glycan" refers to a glycan that includes at least one galactose sugar residue. In some embodiments, a galactosylated glycan is a G1, G2, G1F, G2F, A1, and/or A2 glycan. In some embodiments, a galactosylated glycan includes one or more lactosamine repeats. In some embodiments, a galactosylated glycan is a galactose-alpha-1-3-galactose-containing glycan. In some embodiments, a galactosylated glycan is a tri-antennary glycan or a tetra-antennary glycan. A non-galactosylated glycan includes G0F or G0.

The term "glycoform" is used herein to refer to a particular form of a glycoprotein. That is, when a glycoprotein includes a particular polypeptide that has the potential to be linked to different glycans or sets of glycans, then each different version of the glycoprotein (*i.e*., where the polypeptide is linked to a particular glycan or set of glycans) is referred to as a "glycoform".

"Target glycosylation range", as used herein, refers to a predetermined range of glycosylation levels of one or more particular glycans, such as galactosylated glycans and/or G0F or G0 glycans. In some embodiments, a target glycosylation range is a range of absolute values. In some embodiments, a target glycosylation range is a range of relative values. In some embodiments, a target glycosylation range is a range of one or more particular glycans, such as galactosylated and/or non-galactosylated glycans (*e.g*., G0, G1, G2, G0F, G1F, G2F, A1, A2 or combinations), in a recombinant protein product or described in a predetermined pharmaceutical product specification or a quality control criterion for a pharmaceutical preparation, *e.g.*, a Certificate of Analysis (CofA), a Certificate of Testing (CofT), or a Master Batch Record. In some embodiments, the product specification is a product description in an FDA label, a Physician's Insert, a USP monograph, or an EP monograph.

A target glycosylation range has an upper level (upper value) and a lower level (lower value); the target glycosylation range is the series of glycosylation values between (and including) the upper and lower values. The "target glycosylation range midpoint" means the sum of the upper and lower target glycosylation range levels, divided by 2. In some embodiments, a target glycosylation range or a glycosylation level in general refers to absolute levels of (*e.g*., number of moles of) one or more glycans (*e.g*., non-galactosylated and/or galactosylated glycans (*e.g*., one or more species of galactosylated and/or non-galactosylated glycans)) in a glycoprotein preparation. In some embodiments, a target glycosylation range refers to levels of one or more glycans (*e.g*., non-galactosylated and/or galactosylated glycans (*e.g*., one or more species of galactosylated and/or non-galactosylated glycans)) in a glycoprotein preparation relative to total level of glycans in the glycoprotein preparation. In some embodiments, a target glycosylation range is expressed as "percent" or "percentages", which refers to the number of moles of one or more glycans (*e.g*., Fc glycans) relative to total moles of glycans (*e.g*., Fc glycans) in a recombinant protein preparation.

As used herein, "cell density" refers to the number of cells in a given volume of culture medium. As used herein, "viable cell density" or "VCD" refer to the number of live cells in a given volume of culture medium, as determined by standard viability assays (such as trypan blue dye exclusion method). As used herein, "upper and lower viable cell density levels" refers to the range (values between and including the upper and lower viable cell density levels) of viable cell densities at which use of said cells in a method of the instant claims would yield a recombinant protein having a glycosylation level within a target glycosylation range. The "midpoint" between upper and lower viable cell density levels for a given culture (*e.g*., N-1 VCD or N VCD) means the sum of the values for the upper and lower viable cell density levels for that culture, divided by 2.

As used herein, "feed," "fed," or "feeding" refer to providing a nutritive substance.

As used herein, "coefficient of variation" or "CV" refers to a standard statistical measure of variance and is defined as the standard deviation (*σ*) divided by the mean value (*µ*). The CV can be expressed as a percentage by multiplying by 100.

### Cells

Any host cell that can be used to express a polypeptide of interest (*e.g*., an antibody) can be used in the methods described herein. The cells can be genetically engineered to contain a recombinant nucleic acid sequence (*e.g*., a gene) that encodes a polypeptide of interest (*e.g*., an antibody). For example, useful cells can express a recombinant polypeptide. Recombinant expression of a gene encoding a polypeptide can include construction of an expression vector containing a polynucleotide that encodes the polypeptide. Once a polynucleotide has been obtained, a vector for the production of the polypeptide can be produced by recombinant DNA technology using techniques known in the art. Known methods can be used to construct expression vectors containing polypeptide coding sequences and appropriate transcriptional and translational control signals. These methods include, for example, *in vitro* recombinant DNA techniques, synthetic techniques, and *in vivo* genetic recombination.

An expression vector can be transferred to a host cell by conventional techniques, and the transfected cells can then be cultured by conventional techniques, modified in accordance with the present disclosure, to produce a recombinant polypeptide. A variety of host expression vector systems can be used (*see, e.g.,* U.S. Pat. No. 5,807,715). Such host-expression systems (*e.g*., genetically engineered host expression systems) can be used to produce polypeptides (*e.g*., antibodies) and, where desired, subsequently purified. Such host expression systems include, but are not limited to, yeast (*e.g., Saccharomyces* and *Pichia*) transformed with recombinant yeast expression vectors containing polypeptide coding sequences; insect cell systems infected with recombinant virus expression vectors (*e.g*., baculovirus) containing polypeptide coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing polypeptide coding sequences; or mammalian cell systems (*e.g*., COS, CHO, CHOK1SV, BHK, 293, NS0, 3T3, YB2/0, SP2/0, PERC.6, myeloma, and hybridoma cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In addition, a host cell strain can be chosen that modulates the expression of the inserted sequences, or modifies and processes the gene product in the specific fashion desired. Different host cells have characteristic and specific mechanisms for the post-translational processing and modification of proteins and gene products. Appropriate cell lines or host systems can be chosen to ensure the correct modification and processing of the polypeptide (*e.g*., antibody) expressed. Such cells include, for example, established mammalian cell lines and insect cell lines, animal cells, fungal cells, and yeast cells. Mammalian host cells include, but are not limited to, CHO, Vera, BHK, HeLa, COS, MDCK, HEK-293, NIH-3T3, W138, BT483, Hs578T, HTB2, BT20, T47D, NSO (a murine myeloma cell line that does not endogenously produce any immunoglobulin chains), CRL7030, HsS78Bst cells, PER.C6, SP2/0-Agl4, and hybridoma cells. Additional, nonlimiting examples of animal or mammalian host cells include Chinese hamster ovary cells (CHO), such as CHO-K1 (ATCC CCL-61), DG44 (Chasin et al., 1986, Som. Cell Molec. Genet., 12:555-556; and Kolkekar et al., 1997, Biochem., 36: 10901-10909), CHO- DXB11 (G. Urlaub and L.A. Chasin, 1980 Proc. Natl. Acad. Sci., 77: 4216-4220 . L.H. Graf, and L.A. Chasin 1982, Molec. Cell. Biol, 2: 93-96), CHO-K1 Tet-On cell line (Clontech), CHO designated ECACC 85050302 (CAMR, Salisbury, Wiltshire, UK), CHO clone 13 (GEIMG, Genova, IT), CHO clone B (GEIMG, Genova, IT), CHO-K1/SF designated ECACC 93061607 (CAMR, Salisbury, Wiltshire, UK), RR-CHOK1 designated ECACC 92052129 (CAMR, Salisbury, Wiltshire, UK), CHOKIsv (Edmonds et al., Mol. Biotech. 34: 179-190 (2006)), CHO-S (Pichler et al., Biotechnol. Bioeng. 108:386-94 (2011)), dihydrofolate reductase negative CHO cells (CHO/-DHFR, Urlaub and Chasin, 1980, Proc. Natl. Acad. Sci. USA, 77:4216), and dpl2.CHO cells (U.S. Pat. No. 5,721,121); monkey kidney CV1 cells transformed by SV40 (COS cells, COS-7, ATCC CRL-1651); human embryonic kidney cells (*e.g*., 293 cells, or 293 cells subcloned for growth in suspension culture, Graham et al., 1977, J. Gen. Virol, 36:59); baby hamster kidney cells (BHK, ATCC CCL-10); monkey kidney cells (CVI, ATCC CCL-70); African green monkey kidney cells (VERO-76, ATCC CRL-1587; VERO, ATCC CCL-81); mouse Sertoli cells (TM4, Mather, 1980, Biol. Reprod., 23:243-251); human cervical carcinoma cells (HELA, ATCC CCL-2); canine kidney cells (MDCK, ATCC CCL-34); human lung cells (W138, ATCC CCL-75); human hepatoma cells (HEP-G2, HB 8065); mouse mammary tumor cells (MMT 060562, ATCC CCL-51); buffalo rat liver cells (BRL 3A, ATCC CRL-1442); TRI cells (Mather, 1982, Ann. NY Acad. Sci., 383:44-68); MCR 5 cells; and FS4 cells. Host cells can be engineered to lack the capability of fucosylating a protein, such as an antibody, as described in, for example, US6946292, US7214775, US8679491, and US7763246.

Once a polypeptide described herein (*e.g*., an antibody) has been produced by recombinant expression, it can be purified by any method known in the art for purification, for example, by chromatography (*e.g*., ion exchange, affinity, and sizing column chromatography), centrifugation, differential solubility, or by any other standard technique for the purification of proteins. For example, an antibody can be isolated and purified by appropriately selecting and combining affinity columns such as Protein A column with chromatography columns, filtration, ultrafiltration, salting-out and dialysis procedures (*see, e.g*., Antibodies: A Laboratory Manual, Ed Harlow, David Lane, Cold Spring Harbor Laboratory, 1988). Further, as described herein, a polypeptide (*e.g*., an antibody) can be fused to heterologous polypeptide sequences to facilitate purification. Polypeptides having desired sugar chains can be separated with a lectin column by methods known in the art (*see, e.g*., WO 02/30954).

In accordance with the present disclosure, there may be employed conventional molecular biology, microbiology, and recombinant DNA techniques within the skill of the art. Such techniques are described in the literature (see, *e.g*., Sambrook, Fritsch & Maniatis, Molecular Cloning: A Laboratory Manual, Second Edition (1989) Cold Spring Harbor Laboratory Press, Cold Spring Harbor, N.Y.; DNA Cloning: A Practical Approach, Volumes I and II (D. N. Glover ed. 1985); Oligonucleotide Synthesis (M. J. Gait ed. 1984); Nucleic Acid Hybridization (B. D. Hames & S. J. Higgins eds. (1985)); Transcription And Translation (B. D. Hames & S. J. Higgins, eds. (1984)); Animal Cell Culture (R. I. Freshney, ed. (1986)); Immobilized Cells and Enzymes (IRL Press, (1986)); B. Perbal, A Practical Guide To Molecular Cloning (1984); F. M. Ausubel et al. (eds.), Current Protocols in Molecular Biology, John Wiley & Sons, Inc. (1994).

### Culture Methods

Cells can be cultured in a variety of cell culture media known in the art. Cell culture medium is understood by those of skill in the art to refer to a nutrient solution in which cells, such as animal or mammalian cells, are grown. A cell culture medium generally includes one or more of the following components: an energy source (*e.g*., a carbohydrate such as glucose); amino acids; vitamins; lipids or free fatty acids; and trace elements, *e.g*., inorganic compounds or naturally occurring elements in the micromolar range. Cell culture medium can also contain additional components, such as hormones and other growth factors (*e.g*., insulin, transferrin, epidermal growth factor, serum, and the like); salts (*e.g*., calcium, magnesium and phosphate); buffers (*e.g*., HEPES); nucleosides and bases (*e.g*., adenosine, thymidine, hypoxanthine); antibiotics (*e.g*., gentamycin); and cell protective agents (*e.g*., a Pluronic polyol (Pluronic F68)). In some embodiments, a cell culture medium may include lysine, cysteine, ammonium, manganese, cobalt, putrescine, a peptone, glucose, galactose, galactosamine, glucosamine, glutamine, a lipid (*e.g*., cholesterol), DMSO, and/or dextran sulfate.

Media that has been prepared or is commercially available can be used or modified for utilization in the methods described herein. Nonlimiting examples of such media include Minimal Essential Medium (MEM, Sigma, St. Louis, Mo.); Ham's F10 Medium (Sigma); Dulbecco's Modified Eagles Medium (DMEM, Sigma); RPM 1-1640 Medium (Sigma); HyClone cell culture medium (HyClone, Logan, Utah); Power CH02 (Lonza Inc., Allendale, NJ); and chemically-defined (CD) media, which are formulated for particular cell types, *e.g*., CD-CHO Medium (Invitrogen, Carlsbad, Calif). Culture medium suitable for particular cells being cultured can be determined by a person of ordinary skill in the art without undue experimentation, and such medium can be altered according to the disclosure.

Cell culture conditions (including pH, O₂, CO₂, agitation rate and temperature) suitable for cellular production of polypeptides described herein (*e.g*., antibodies) are those that are known in the art, such as conditions for batch, continuous, or fed-batch culturing of cells. For example, pH of cell culture medium is generally maintained at about 6.8 to about 7.2.

In some embodiments, cells are cultured in one or more stages. For example, a culture method can include a first stage (*e.g*., using a first medium) and a second stage (e.g., using a second medium). In some embodiments, a culture method includes more than two stages, *e.g*., 3, 4, 5, 6, or more stages. A culture method can be 5, 6, 7, 8, 9, 10, 11, 12, 13, 14, 15, 16, 17, 18, 19, 20, or more days. In some embodiments, a culture method includes at least two stages. For example, a first stage can include culturing cells to a first viable cell density, and a second stage can include culturing cells to a second viable cell density.

Typically the cell cultures that precede the final production culture are used to generate the seed cells that will be used to inoculate the production bioreactor. The preceding cell cultures are referred to as N-x to N-1 cultures, where N-1 refers to the culture just prior to the final production culture (the N culture). The seed cell density can have a positive impact on the level of recombinant protein produced. Product levels tend to increase with increasing seed density. Improvement in titer is tied not only to higher seed density, but is likely to be influenced by the metabolic and cell cycle state of the cells that are placed into production. Surprisingly, glycosylation is also tied to seed density, and inappropriate selection of seed density can cause adverse consequences on glycosylation.

Seed cells can be produced by any culture method. An N-1 bioreactor can be run to provide cells at a desired density to inoculate a production bioreactor. The N-1 stage may be used to grow cells to densities of about 10 x 10⁵ to about 75 x 10⁵ cells/mL. The N-1 bioreactor can be used to generate bolus seed cultures or can be used as a rolling seed stock culture that could be maintained to seed multiple production bioreactors at selected seed cell density. The duration of the growth stage of production can range from 7 to 14 days and can be designed so as to maintain cells in exponential growth prior to inoculation of the production bioreactor. Perfusion rates, medium formulation and timing are optimized to grow cells and deliver them to the production bioreactor in a state that is most conducive to optimizing their production. Seed cell densities of 10 x 10⁵ to about 75 x 10⁵ cells/mL can be achieved for seeding production bioreactors. Higher seed cell densities at inoculation can cause increased variation of glycosylation levels between batches of recombinant glycoprotein.

In general, cell culture methods are classified as batch culture, continuous culture, and fed-batch culture. Any of these culture methods can be used to grow cells that produce polypeptides (*e.g*., antibodies) having targeted levels of glycans (*e.g*., galactosylated glycans and/or non-galactosylated glycans such as G0 or G0F glycans).

In batch culture, a small amount of seed culture solution is added to a medium and cells are grown without any addition of a new medium or discharge of culture solution during culture. Continuous culture is a culture method in which a medium is added and discharged continuously during culture. This continuous method includes perfusion culture. Fed-batch culture is a method between batch culture and continuous culture. In a fed-batch culture, a cell culture is fed or supplemented continuously or sequentially during culture, but unlike continuous culture, discharge of culture solution is not carried out during culture.

According to the present disclosure, cell culture can be carried out under conditions for large or small scale production of polypeptides (*e.g*., antibodies), using culture vessels and/or culture apparatuses that are conventionally employed for animal or mammalian cell culture. For example, tissue culture dishes, T-flasks, shaker flasks, and spinner flasks can be used on a laboratory scale. For culturing on a larger scale (*e.g*., 1 L, 10 L, 100 L, 500 L, 5000 L, or more), a fluidized bed bioreactor, a hollow fiber bioreactor, a roller bottle culture, or a stirred tank bioreactor system can be used (*e.g*., as described in U.S. Pat. Nos. 7,541,164 and 7,332,303).

In particular methods, levels of one or more glycans (*e.g*., galactosylated glycans and/or non-galactosylated glycans such as G0 or G0F glycans) in a preparation of polypeptides (*e.g*., antibodies) are monitored during one or more times (*e.g*., one or more stages) of cell culture, thereby allowing adjustment or termination of the culture in order, for example, to achieve a target level of polypeptides (*e.g*., antibodies) having targeted levels of glycans (*e.g*., galactosylated glycans and/or non-galactosylated glycans such as G0 or G0F glycans).

### Polypeptides

Described herein are therapeutic preparations of polypeptides (*e.g*., glycoproteins), and methods of making and using such preparations. Glycoproteins include, for example, any of a variety of hematologic agents (including, for instance, erythropoietin, blood-clotting factors, etc.), interferons, colony stimulating factors, antibodies, enzymes, and hormones. The identity of a particular glycoprotein is not intended to limit the present disclosure, and a therapeutic preparation described herein can include any glycoprotein of interest, *e.g*., a glycoprotein having an Fc region.

A glycoprotein described herein can include a target-binding domain that binds to a target of interest (*e.g*., binds to an antigen). For example, a glycoprotein, such as an antibody, can bind to a transmembrane polypeptide *(e.g.,* receptor) or ligand *(e.g.,* a growth factor). Exemplary molecular targets (*e.g*., antigens) for glycoproteins described herein (*e.g*., antibodies) include CD proteins such as CD2, CD3, CD4, CD8, CD11, CD19, CD20, CD22, CD25, CD33, CD34, CD40, CD52; members of the ErbB receptor family such as the EGF receptor (EGFR, HER1, ErbBI), HER2 (ErbB2), HER3 (ErbB3) or HER4 (ErbB4) receptor; macrophage receptors such as CRIg; tumor necrosis factors such as TNFa or TRAIL/Apo-2; cell adhesion molecules such as LFA-1, Macl, p150,95, VLA-4, ICAM-1, VCAM and αvβ3 integrin including either a or β subunits thereof (*e.g*., anti-CD11a, anti-CD18 or anti-CD11b antibodies); growth factors and receptors such as EGF, FGFR (*e.g*., FGFR3) and VEGF; IgE; cytokines such as IL1; cytokine receptors such as IL2 receptor; blood group antigens; flk2/flt3 receptor; obesity (OB) receptor; mpl receptor; CTLA-4; protein C; neutropilins; ephrins and receptors; netrins and receptors; slit and receptors; chemokines and chemokine receptors such as CCL5, CCR4, CCR5; amyloid beta; complement factors, such as complement factor D; lipoproteins, such as oxidized LDL (oxLDL); lymphotoxins, such as lymphotoxin alpha (LTa). Other molecular targets include Tweak, B7RP-1, proprotein convertase subtilisin/kexin type 9 (PCSK9), sclerostin, c-kit, Tie-2, c-fms, and anti-M1.

In some embodiments, described herein are therapeutic polypeptides (*e.g.*, glycoprotein) having targeted levels of glycans (*e.g*., galactosylated glycans and/or non-galactosylated glycans such as G0 or G0F glycans), where the targeted levels are the levels of glycans (*e.g*., galactosylated glycans and/or non-galactosylated glycans such as G0 or G0F glycans) in a reference polypeptide product (*e.g*., glycoprotein product). Nonlimiting, exemplary glycoprotein products can include abatacept (ORENCIA®, Bristol-Myers Squibb), abciximab (REOPRO®, Roche), adalimumab (HUMIRA®, Bristol-Myers Squibb), aflibercept (EYLEA®, Regeneron Pharmaceuticals), alefacept (AMEVIVE®, Astellas Pharma), alemtuzumab (CAMPATH®, Genzyme/Bayer), basiliximab (SIMULECT®, Novartis), belatacept (NULOJIX®, Bristol- Myers Squibb), belimumab (BENLYSTA®, Glaxo SmithKline), bevacizumab (AVASTIN®, Roche), canakinumab (ILARIS®, Novartis), brentuximab vedotin (ADCETRIS®, Seattle Genetics), certolizumab (CIMZIA®, UCB, Brussels, Belgium), cetuximab (ERBITUX®, Merck-Serono), daclizumab (ZENAPAX®, Hoffmann-La Roche), denileukin diftitox (ONTAK®, Eisai), denosumab (PROLIA®, Amgen; XGEVA®, Amgen), eculizumab (SOLIRIS®, Alexion Pharmaceuticals), efalizumab (RAPTIVA®, Genentech), etanercept (ENBREL®, Amgen-Pfizer), gemtuzumab (MYLOTARG®, Pfizer), golimumab (SIMPONI®, Janssen), ibritumomab (ZEVALIN®, Spectrum Pharmaceuticals), infliximab (REMICADE®, Centocor), ipilimumab (YERVOY™, Bristol-Myers Squibb), muromonab (ORTHOCLONE OKT3®, Janssen-Cilag), natalizumab (TYSABRI®, Biogen Idee, Elan), ofatumumab (ARZERRA®, Glaxo SmithKline), omalizumab (XOLAIR®, Novartis), palivizumab (SYNAGIS®, Medlmmune), panitumumab (VECTIBIX®, Amgen), ranibizumab (LUCENTIS®, Genentech), rilonacept (ARCALYST®, Regeneron Pharmaceuticals), rituximab (MABTHERA®, Roche), tocilizumab (ACTEMRA®, Genentech; RoActemra, Hoffman-La Roche), tositumomab (BEXXAR®, Glaxo SmithKline), and trastuzumab (HERCEPTIN®, Roche).

In some embodiments, a level of one or more glycans (*e.g*., galactosylated glycans and/or non-galactosylated glycans such as G0 or G0F glycans) in a polypeptide product is determined by analyzing one or more preparations (*e.g*., one or more lots) of the polypeptide. In some embodiments, a level of one or more glycans (*e.g*., galactosylated glycans and/or non-galactosylated glycans such as G0 or G0F glycans) in a polypeptide product is a range of the one or more glycans in two or more preparations of the polypeptide (*e.g*., two or more lots of the polypeptide product). In some embodiments, a level of one or more glycans is a range (*e.g*., spanning a lowest level of the one or more glycans to a highest level of the one or more glycans) in two or more lots of the polypeptide product.

### Pharmaceutical Compositions and Administration

A recombinant protein, such as the glycoproteins described herein, can be incorporated (*e.g*., formulated) into a pharmaceutical composition. Accordingly, in another aspect, the present invention relates to a method for producing a pharmaceutical composition comprising a recombinant protein having a reduced inter-batch glycosylation level variability, which includes the step wherein said method comprises carrying out the cell culturing method for reducing the variability of glycosylation levels between batches of said recombinant protein as described herein.

In some embodiments, the method for manufacture of a pharmaceutical composition further comprises combining said recombinant protein having a reduced inter-batch glycosylation level variability (*i.e*., obtained from the cell culturing method described herein) together with a pharmaceutically acceptable carrier. Optionally, the method further includes lyophilization of said pharmaceutical composition together with the pharmaceutically acceptable carrier, as is generally known in the art.

Such a pharmaceutical composition is useful as an alternative and/or improved composition for the prevention and/or treatment of one or more diseases relative to a corresponding reference glycoprotein. Pharmaceutical compositions comprising a glycoprotein can be formulated by methods known to those skilled in the art. The pharmaceutical composition can be administered parenterally in the form of an injectable formulation comprising a sterile solution or suspension in water or another pharmaceutically acceptable liquid. For example, the pharmaceutical composition can be formulated by suitably combining the glycoprotein with pharmaceutically acceptable vehicles or media, such as sterile water and physiological saline, vegetable oil, emulsifier, suspension agent, surfactant, stabilizer, flavoring excipient, diluent, vehicle, preservative, binder, followed by mixing in a unit dose form required for generally accepted pharmaceutical practices. The amount of active ingredient included in the pharmaceutical preparations is such that a suitable dose within the designated range is provided.

A sterile composition for injection can be formulated in accordance with conventional pharmaceutical practices using distilled water for injection as a vehicle. For example, physiological saline or an isotonic solution containing glucose and other supplements such as D- sorbitol, D-mannose, D-mannitol, and sodium chloride may be used as an aqueous solution for injection, optionally in combination with a suitable solubilizing agent, for example, alcohol such as ethanol and polyalcohol such as propylene glycol or polyethylene glycol, and a nonionic surfactant such as polysorbate 80™, HCO-50 and the like.

Nonlimiting examples of oily liquid include sesame oil and soybean oil, and it may be combined with benzyl benzoate or benzyl alcohol as a solubilizing agent. Other items that may be included are a buffer such as a phosphate buffer, or sodium acetate buffer, a soothing agent such as procaine hydrochloride, a stabilizer such as benzyl alcohol or phenol, and an antioxidant. A formulated injection can be packaged in a suitable ampule.

Route of administration can be parenteral, for example, administration by injection, transnasal administration, transpulmonary administration, or transcutaneous administration. Administration can be systemic or local by intravenous injection, intramuscular injection, intraperitoneal injection, subcutaneous injection.

A suitable means of administration can be selected based on the age and condition of the patient. A single dose of the pharmaceutical composition containing a modified glycoprotein can be selected from a range of 0.001 to 1000 mg/kg of body weight. On the other hand, a dose can be selected in the range of 0.001 to 100000 mg/body weight, but the present disclosure is not limited to such ranges. The dose and method of administration varies depending on the weight, age, condition, and the like of the patient, and can be suitably selected as needed by those skilled in the art.

The disclosure is further illustrated by the following example. The examples are provided for illustrative purposes only. They are not to be construed as limiting the scope or content of the disclosure in any way.

### EXAMPLE

### Effect of N-1 VCD & N VCD on Glycosylation

### Materials and Methods:

A monoclonal antibody manufacturing process started with a vial thaw of the NSO working cell bank (WCB) in a suitable cell culture medium. The cells were expanded initially in shake flasks and shaking roller bottles in an incubator with temperature maintained between 30.0 and 38.0°C. Further inoculum expansion took place in a 20 L bioreactor, followed by scale up into a 100 L stirred tank bioreactor. The 20 L culture was grown at a temperature between 30.0 and 38.0°C. The 100 L bioreactor culture was maintained for multiple passages by removing a portion of culture and re-feeding the remainder of culture with fresh media. The culture removed was used to inoculate the 100 L or 1,000 L bioreactor, or was discarded.

The 1,000 L bioreactor culture in turn seeded a 4,000 L bioreactor, and this culture was ultimately used to inoculate a 20,000 L stirred tank production bioreactor. The production bioreactor was operated in fed-batch mode. All bioreactor cultures from 100 L to 20,000 L were operated at a temperature between 30.0 and 38.0°C, dissolved oxygen concentrations between 5.0 and 50.0%, and pH between 6.80 and 7.50.

The 4,000 L bioreactor culture is the "N-1 culture," and the 20,000 L culture is the "N culture". The N-1 culture in the 4,000 L bioreactor was cultured to a viable cell density (VCD) that was between 80% and 120% of the midpoint between N-1 VCD upper and lower levels, within which range the 4,000 L bioreactor culture was considered a "constrained seed culture." If the N-1 culture VCD was not between 80% and 120% of the midpoint between the N-1 VCD upper and lower levels, then the seed culture was considered an "unconstrained culture." Preferably, once the N-1 culture reached the appropriate VCD level for a constrained seed culture, it was used to inoculate the 20,000 L bioreactor (the N culture), to make a constrained production culture.

The constrained production culture in the 20,000 L bioreactor was not fed until the VCD reached a level between the predetermined N VCD midpoint level and N VCD lower level, after which the constrained production culture was fed (*e.g.*, with fresh medium, and/or supplements) If the VCD of the production culture was not between the N VCD midpoint and lower levels, or if the production culture was inoculated with an unconstrained seed culture, then the production culture was considered an "unconstrained culture." Comparisons of constrained and unconstrained cultures are shown in TABLE 1.

After 13 to 14 days' culture in the 20,000 L bioreactor, the bioreactor contents were purified by protein A and ion exchange chromatography, via methods disclosed in US7847071B2.

The percent glycosylation of antibody was measured using a High Performance Liquid Chromatography method followed by Fluorescence detection. N-linked oligosaccharides were released by incubation with recombinant glycosidase. The proteins were precipitated and removed, and the released oligosaccharides were then hydrolyzed. The oligosaccharides were labelled with a fluorescent agent for HPLC analysis. The percent glycosylation was correlated to the observed fluorescence level.

The upper and lower viable cell density (VCD) levels for any given cell are determined empirically by measuring VCD (*e.g*., of the N-1 and N cultures used to produce a recombinant protein), measuring the glycosylation profiles of the protein produced from those cultures, and correlating VCD data with the glycosylation data. These steps permit determination of "upper and lower viable cell density levels," which define the range of VCDs at which the use of said cell for production of the protein would yield a protein having a glycosylation level within a target glycosylation range.

### Results:

Using the methods described above for constrained and unconstrained cultures, the %GOF, %G1F and %G2F values were obtained for a recombinant protein having upper and lower target glycosylation ranges for G0F, G1F, and G2F. As shown in TABLE 1 below, the constrained culture conditions yielded a reduction in variability for G0F, G1F and G2F as indicated by the range of % glycosylation spanned. The minimum and maximum values of % glycosylation for Unconstrained and Constrained Production Batches are shown in Table 1:

**Table 1. Effect of N-1 VCD & N VCD on Glycosylation**

| | % G0F min/max (%CV) | % G1F min/max (%CV) | % G2F min/max (%CV) |
|---|---|---|---|
| Target Range | 32.0 - 49.0 | 28.0 - 35.0 | 6.0 - 13.0 |
| Unconstrained | 35.6 - 48.8 (7.3) | 28.0 - 33.7 (4.6) | 7.2 - 11.0 (10.8) |
| Constrained | 39.8 - 42.8 (2.3) | 30.2 - 32.0 (1.8) | 8.5 - 9.7 (4.3) |

It will be understood that each of the elements described above, or two or more together may also find a useful application in other types of methods differing from the type described above. Without further analysis, the foregoing will so fully reveal the gist of the present disclosure that others can, by applying current knowledge, readily adapt it for various applications without omitting features that, from the standpoint of prior art, fairly constitute essential characteristics of the generic or specific aspects of this disclosure set forth in the appended claims. The foregoing embodiments are presented by way of example only; the scope of the present disclosure is to be limited only by the following claims.

## Claims

1. A method of reducing the variability of glycosylation levels between batches of a recombinant protein having a target glycosylation range, and said target glycosylation range having a midpoint, the method comprising:
(a) providing a cell suitable for expressing said recombinant protein;
(b) determining upper and lower viable cell density levels for a seed culture of said cells (N-1 VCD), determining upper and lower viable cell density levels for a production culture of said cells (N VCD), and determining the midpoint between said N-1 VCD upper and lower levels, and the midpoint between said N VCD upper and lower levels,
wherein the upper and lower viable cell density levels are the range, i.e.,values between and including the upper and lower viable cell density levels, of viable cell densities at which recombinant protein having a glycosylation level within the target glycosylation range is obtained;
(c) culturing said cells to a viable cell density which is between about 80% and about 120% of the midpoint between said N-1 VCD upper and lower levels, thereby producing a constrained seed culture; and
(d) inoculating a culture medium with said constrained seed culture to make a constrained production culture, culturing said constrained production culture to a viable cell density between said N VCD midpoint and lower levels, and then feeding said constrained production culture;
wherein steps (c) and (d) yield a first batch of said recombinant protein from said cells, wherein said glycosylation level of said recombinant protein is within said target glycosylation range;
(e) repeating steps (c) through (d) to yield at least one subsequent batch of said recombinant protein from said cells, wherein the glycosylation levels of said first and at least one subsequent batch are between about 75% to about 125% of the target glycosylation range midpoint, and/or the coefficient of variation of the glycosylation levels of said recombinant proteins of said first and at least one subsequent batch is 4.5% or less, wherein the coefficient of variation is the standard deviation (σ) divided by the mean value (µ).

2. The method of claim 1, wherein said cell suitable for expressing said recombinant protein is a eukaryotic cell.

3. The method of claim 2, wherein said cell is a fungal cell, a plant cell, an insect cell, or a mammalian cell.

4. The method of claim 3, wherein said cell is a mammalian cell.

5. The method of claim 4, wherein said cell is selected from the group consisting of CHO, CHOK1SV, NS0, SP2/0, PERC.6, myeloma, and hybridoma cells.

6. The method of any one of claims 1-5, wherein said N-1 VCD is from about 10 x 10⁵ to about 75 x 10⁵ cells/mL and said N VCD is from about 10 x 10⁵ to about 50 x 10⁵ cells/mL.

7. The method of any one of claims 1-5, wherein said N-1 VCD is from about 10 x 10⁵ to about 50 x 10⁵ cells/mL and said N VCD is from about 10 x 10⁵ to about 30 x 10⁵ cells/mL.

8. The method of any one of claims 1-5, wherein said N-1 VCD is from about 10 x 10⁵ to about 25 x 10⁵ cells/mL and said N VCD is from about 10 x 10⁵ to about 20 x 10⁵ cells/mL.

9. The method of any one of claims 1-5, wherein said N-1 VCD is from about 15 x 10⁵ to about 25 x 10⁵ cells/mL and said N VCD is from about 15 x 10⁵ to about 20 x 10⁵ cells/mL.

10. The method of any one of claims 1-9, wherein said feeding of said constrained production culture is after about 24 and 108 hours following said inoculating with said constrained seed culture.

11. The method of any one of claims 1-10, wherein said glycosylation level is determined on the basis of the content of a glycan selected from the group consisting of G0F, G1F, G2F, and combinations thereof.

12. The method of any one of claims 1-10, wherein said glycosylation level is determined on the basis of the content of a glycan selected from the group consisting of G0, G1, G2, and combinations thereof.

13. The method of any one of claims 1-11, wherein:
(a) glycan G0F comprises from about 10% to about 80% of said glycosylation;
(b) glycan G1F comprises from about 10% to about 80% of said glycosylation; and
(c) glycan G2F comprises from about 10% to about 80% of said glycosylation.

14. The method of any one of claims 1-10 and 12, wherein:
(a) glycan G0 comprises from about 10% to about 80% of said glycosylation;
(b) glycan G1 comprises from about 10% to about 80% of said glycosylation; and
(c) glycan G2 comprises from about 10% to about 80% of said glycosylation.

15. The method of any one of claims 1-14, wherein said target glycosylation range is a predetermined pharmaceutical product specification or a quality control criterion.

16. The method of any one of claims 1-15, wherein the target glycosylation range is a glycosylation range of one or more glycans in a preparation of a recombinant protein.

17. A method for producing a pharmaceutical composition comprising a recombinant protein having a reduced inter-batch glycosylation level variability, wherein said method comprises reducing the variability of glycosylation levels between batches of said recombinant protein by carrying out a method according to any one of claims 1 to 16.

18. The method of claim 17, further comprising combining said recombinant protein having a reduced inter-batch glycosylation level variability together with a pharmaceutically acceptable carrier, and, optionally, subjecting the pharmaceutical composition with the pharmaceutically acceptable carrier to lyophilization.

19. The method of any one of claims 1-18, wherein said recombinant protein is a recombinant therapeutic protein.

20. The method of any one of claims 1-19, wherein said recombinant protein is a recombinant therapeutic antibody.

21. The method of any one of claims 1-20, wherein the recombinant therapeutic antibody is selected from the group consisting of abatacept, abciximab, adalimumab, aflibercept, alefacept, alemtuzumab, basiliximab, belatacept, belimumab, bevacizumab, canakinumab, brentuximab vedotin, certolizumab, cetuximab, daclizumab, denileukin diftitox, denosumab, eculizumab, efalizumab, etanercept, gemtuzumab, golimumab, ibritumomab, infliximab, ipilimumab, muromonab, natalizumab, ofatumumab, omalizumab, palivizumab, panitumumab, ranibizumab, rilonacept, rituximab, tocilizumab, tositumomab, and trastuzumab.

## Patentansprüche

1. Verfahren zum Reduzieren der Variabilität der Glykosylierungsniveaus zwischen Chargen eines rekombinanten Proteins mit einem Zielglykosylierungsbereich und einem Mittelpunkt des Zielglykosylierungsbereichs, wobei das Verfahren umfasst:
(a) Bereitstellen einer Zelle, die geeignet ist, das rekombinante Protein zu exprimieren;
(b) Bestimmen der oberen und unteren lebensfähigen Zelldichte für eine Samenkultur der Zellen (N-1 VCD), Bestimmen der oberen und unteren lebensfähigen Zelldichte für eine Produktionskultur der Zellen (N VCD) und Bestimmen des Mittelpunktes zwischen der oberen und unteren N-1 VCD-Grenze und des Mittelpunktes zwischen den oberen und unteren N VCD-Grenze,
wobei die oberen und unteren lebensfähigen Zelldichtewerte der Bereich, d.h. Werte zwischen und einschließlich der oberen und unteren lebensfähigen Zelldichtewerte, von lebensfähigen Zelldichten sind, bei denen rekombinantes Protein mit einem Glykosylierungsgrad innerhalb des Zielglykosylierungsbereichs erhalten wird;
(c) Kultivieren der Zellen zu einer lebensfähigen Zelldichte, die zwischen etwa 80% und etwa 120% des Mittelpunktes zwischen dem oberen und unteren N-1 VCD-Spiegel liegt, wodurch eine eingeschränkte Samenkultur erzeugt wird; und
(d) Impfen eines Kulturmediums mit der eingeschränkten Samenkultur, um eine eingeschränkte Produktionskultur herzustellen, Kultivieren der eingeschränkten Produktionskultur auf eine lebensfähige Zelldichte zwischen dem N-VCD-Mittelpunkt und niedrigeren Niveaus, und dann Nährung der eingeschränkten Produktionskultur;
wobei die Schritte (c) und (d) eine erste Charge des rekombinanten Proteins aus den Zellen ergeben, wobei der Glykosylierungsgrad des rekombinanten Proteins innerhalb des Zielglykosylierungsbereichs liegt;
(e) Wiederholen der Schritte (c) bis (d), um mindestens eine nachfolgende Charge des rekombinanten Proteins aus den Zellen zu erhalten, wobei die Glykosylierungsniveaus der ersten und mindestens einer nachfolgenden Charge zwischen etwa 75% und etwa 125% des Mittelwerts des Zielglykosylierungsbereichs liegen, und/oder der Variationskoeffizient der Glykosylierungsniveaus der rekombinanten Proteine der ersten und mindestens einer nachfolgenden Charge 4,5% oder weniger beträgt, wobei der Variationskoeffizient die Standardabweichung (σ) dividiert durch den Mittelwert (µ) ist.

2. Verfahren nach Anspruch 1, worin die Zelle, die zur Expression des rekombinanten Proteins geeignet ist, eine eukaryotische Zelle ist.

3. Verfahren nach Anspruch 2, worin die Zelle eine Pilzzelle, eine Pflanzenzelle, eine Insektenzelle oder eine Säugetierzelle ist.

4. Verfahren nach Anspruch 3, worin die Zelle eine Säugetierzelle ist.

5. Verfahren nach Anspruch 4, worin die Zelle ausgewählt ist aus der Gruppe bestehend aus CHO, CHOK1SV, NS0, SP2/0, PERC.6, Myelom- und Hybridomzellen.

6. Verfahren nach einem der Ansprüche 1-5, worin der N-1 VCD von etwa 10 x 10⁵ bis etwa 75 x 10⁵ Zellen/mL und der N VCD von etwa 10 x 10⁵ bis etwa 50 x 10⁵ Zellen/mL beträgt.

7. Verfahren nach einem der Ansprüche 1-5, worin der N-1 VCD von etwa 10 x 10⁵ bis etwa 50 x 10⁵ Zellen/mL und der N VCD von etwa 10 x 10⁵ bis etwa 30 x 10⁵ Zellen/mL beträgt.

8. Verfahren nach einem der Ansprüche 1-5, worin der N-1 VCD von etwa 10 x 10⁵ bis etwa 25 x 10⁵ Zellen/mL und der N VCD von etwa 10 x 10⁵ bis etwa 20 x 10⁵ Zellen/mL beträgt.

9. Verfahren nach einem der Ansprüche 1-5, worin der N-1 VCD von etwa 15 x 10⁵ bis etwa 25 x 10⁵ Zellen/mL und der N VCD von etwa 15 x 10⁵ bis etwa 20 x 10⁵ Zellen/mL beträgt.

10. Verfahren nach einem der Ansprüche 1-9, worin die Nährung der eingeschränkten Produktionskultur etwa 24 und 108 Stunden nach dem Impfen mit der eingeschränkten Samenkultur erfolgt.

11. Verfahren nach einem der Ansprüche 1-10, wobei der Glykosylierungsgrad auf der Grundlage des Gehalts eines Glykans bestimmt wird, das aus der Gruppe ausgewählt ist, die aus G0F, G1F, G2F und Kombinationen derselben besteht.

12. Verfahren nach einem der Ansprüche 1-10, wobei der Glykosylierungsgrad auf der Grundlage des Gehalts an einem Glycan bestimmt wird, das aus der Gruppe ausgewählt ist, die aus G0, G1, G2 und Kombinationen derselben besteht.

13. Verfahren nach einem der Ansprüche 1-11, wobei:
(a) Glykan G0F umfasst von etwa 10% bis etwa 80% der Glykosylierung;
(b) Glykan G1F umfasst von etwa 10% bis etwa 80% der Glykosylierung; und
(c) Glykan G2F umfasst von etwa 10% bis etwa 80% der Glykosylierung.

14. Verfahren nach einem der Ansprüche 1-10 und 12, wobei:
(a) Glykan G0 umfasst von etwa 10% bis etwa 80% der Glykosylierung;
(b) Glykan G1 etwa 10% bis etwa 80% der Glykosylierung umfasst; und
(c) Glykan G2 umfasst von etwa 10% bis etwa 80% der Glykosylierung.

15. Verfahren nach einem der Ansprüche 1-14, worin der Zielglykosylierungsbereich eine vorgegebene pharmazeutische Produktspezifikation oder ein Qualitätskontrollkriterium ist.

16. Verfahren nach einem der Ansprüche 1-15, worin der Zielglykosylierungsbereich ein Glykosylierungsbereich von einem oder mehreren Glykanen ist, in einer Zubereitung eines rekombinanten Proteins.

17. Verfahren zur Herstellung einer pharmazeutischen Zusammensetzung, umfassend ein rekombinantes Protein mit einer reduzierten Variabilität des Glykosylierungsniveaus zwischen den Chargen des rekombinanten Proteins, wobei das Verfahren das Reduzieren der Variabilität des Glykosylierungsniveaus zwischen den Chargen des rekombinanten Proteins durch Ausführen eines Verfahrens nach einem der Ansprüche 1 bis 16 umfasst.

18. Verfahren nach Anspruch 17, ferner umfassend das Kombinieren des rekombinanten Proteins mit einer reduzierten Variabilität des Glykosylierungsniveaus zwischen den Chargen zusammen mit einem pharmazeutisch akzeptablen Träger und gegebenenfalls das Unterwerfen der pharmazeutischen Zusammensetzung mit dem pharmazeutisch akzeptablen Träger einer Lyophilisierung.

19. Verfahren nach einem der Ansprüche 1-18, worin das rekombinante Protein ein rekombinantes therapeutisches Protein ist.

20. Verfahren nach einem der Ansprüche 1-19, worin das rekombinante Protein ein rekombinanter therapeutischer Antikörper ist.

21. Verfahren nach einem der Ansprüche 1-20, worin der rekombinante therapeutische Antikörper ausgewählt ist aus der Gruppe bestehend aus Abatacept, Abciximab, Adalimumab, Aflibercept, Alefacept, Alemtuzumab, Basiliximab, Belatacept, Belimumab, Bevacizumab, Canakinumab, Brentuximab Vedotin, Certolizumab, Cetuximab, Daclizumab, Denileukin Diftitox, Denosumab, Eculizumab, Efalizumab, Etanercept, Gemtuzumab, Golimumab, Ibritumomab, Infliximab, Ipilimumab, Muromonab, Natalizumab, Ofatumumab, Omalizumab, Palivizumab, Panitumumab, Ranibizumab, Rilonacept, Rituximab, Tocilizumab, Tositumomab und Trastuzumab.

## Revendications

1. Procédé de réduction de la variabilité des niveaux de glycosylation entre des lots d'une protéine recombinante ayant une plage de glycosylation cible, et ladite plage de glycosylation cible ayant un point milieu, le procédé comprenant :
(a) fournir une cellule appropriée pour exprimer ladite protéine recombinante ;
(b) déterminer les niveaux supérieur et inférieur de densité cellulaire viable pour une culture de semence desdites cellules (N-1 VCD), déterminer les niveaux supérieur et inférieur de densité cellulaire viable pour une culture de production desdites cellules (N VCD), et déterminer le point médian entre lesdits niveaux supérieur et inférieur de N-1 VCD et le point médian entre lesdits niveaux supérieur et inférieur de N VCD,
dans laquelle les niveaux de densité cellulaire viable supérieur et inférieur sont la plage, c'est-à-dire les valeurs comprises entre les niveaux de densité cellulaire viable supérieur et inférieur, de densités cellulaires viables à partir desquelles une protéine recombinante ayant un niveau de glycosylation dans la plage de glycosylation cible est obtenue ;
(c) cultiver lesdites cellules jusqu'à une densité cellulaire viable qui se situe entre environ 80 % et environ 120 % du point médian entre lesdits niveaux supérieur et inférieur de N-1 VCD, produisant ainsi une culture de semences contrainte ; et
(d) l'inoculation d'un milieu de culture avec ladite culture de semence contrainte pour faire une culture de production contrainte, la culture de ladite culture de production contrainte à une densité cellulaire viable entre ledit point milieu N VCD et les niveaux inférieurs, puis l'alimentation de ladite culture de production contrainte ;
dans laquelle les étapes (c) et (d) produisent un premier lot de ladite protéine recombinante à partir desdites cellules, dans laquelle ledit niveau de glycosylation de ladite protéine recombinante est dans ladite plage de glycosylation cible ;
(e) répéter les étapes (c) à (d) pour obtenir au moins un lot ultérieur de ladite protéine recombinante à partir desdites cellules, dans lequel les niveaux de glycosylation dudit premier lot et d'au moins un lot ultérieur sont compris entre environ 75 % et environ 125 % du point milieu de l'intervalle de glycosylation cible, et/ou le coefficient de variation des niveaux de glycosylation desdites protéines recombinantes dudit premier lot et au moins un lot ultérieur est 4,5 % ou moins, le coefficient de variation représentant l'écart type (σ) divisé par la valeur moyenne (µ).

2. Procédé selon la revendication 1, dans lequel ladite cellule appropriée pour exprimer ladite protéine recombinante est une cellule eucaryote.

3. Procédé selon la revendication 2, dans lequel ladite cellule est une cellule fongique, une cellule végétale, une cellule insecte ou une cellule mammifère.

4. Procédé selon la revendication 3, dans lequel ladite cellule est une cellule mammifère.

5. Procédé selon la revendication 4, dans lequel ladite cellule est choisie dans le groupe constitué des cellules CHO, CHOK1SV, NS0, SP2/0, PERC.6, myélome et hybridomes.

6. Procédé selon l'une quelconque des revendications 1 à 5, dans lequel ledit N-1 VCD est d'environ 10 x 10⁵ à environ 75 x 10⁵ cellules/mL et ledit N VCD est d'environ 10 x 10⁵ à environ 50 x 10⁵ cellules/mL.

7. Procédé selon l'une quelconque des revendications 1 à 5, dans lequel ledit N-1 VCD est d'environ 10 x 10⁵ à environ 50 x 10⁵ cellules/mL et ledit N VCD est d'environ 10 x 10⁵ à environ 30 x 10⁵ cellules/mL.

8. Procédé selon l'une quelconque des revendications 1 à 5, dans lequel ledit N-1 VCD est d'environ 10 x 10⁵ à environ 25 x 10⁵ cellules/mL et ledit N VCD est d'environ 10 x 10⁵ à environ 20 x 10⁵ cellules/mL.

9. Procédé selon l'une quelconque des revendications 1 à 5, dans lequel ledit N-1 VCD est d'environ 15 x 10⁵ à environ 25 x 10⁵ cellules/mL et ledit N VCD est d'environ 15 x 10⁵ à environ 20 x 10⁵ cellules/mL.

10. Procédé selon l'une quelconque des revendications 1 à 9, dans lequel ladite alimentation de ladite culture de production contrainte est après environ 24 et 108 heures après ladite inoculation avec ladite culture de semences contrainte.

11. Procédé selon l'une quelconque des revendications 1 à 10, dans lequel ledit niveau de glycosylation est déterminé sur la base de la teneur d'un glycane choisi dans le groupe constitué par G0F, G1F, G2F et leurs combinaisons.

12. Procédé selon l'une quelconque des revendications 1 à 10, dans lequel ledit niveau de glycosylation est déterminé sur la base de la teneur d'un glycane choisi dans le groupe constitué de G0, G1, G2 et de leurs combinaisons.

13. Procédé de l'une quelconque des revendications 1-11, dans lequel :
(a) le glycan G0F comprend d'environ 10 % à environ 80 % de ladite glycosylation ;
(b) le glycan G1F comprend d'environ 10 % à environ 80 % de ladite glycosylation ; et
(c) le glycan G2F comprend d'environ 10 % à environ 80 % de ladite glycosylation.

14. Procédé de l'une quelconque des revendications 1-10 et 12, dans lequel :
(a) le glycan G0 comprend d'environ 10 % à environ 80 % de ladite glycosylation ;
(b) le glycan G1 comprend d'environ 10 % à environ 80 % de ladite glycosylation ; et
(c) le glycan G2 comprend d'environ 10 % à environ 80 % de ladite glycosylation.

15. Procédé selon l'une quelconque des revendications 1-14, dans lequel ladite plage de glycosylation cible est une spécification de produit pharmaceutique prédéterminée ou un critère de contrôle de qualité.

16. Procédé selon l'une quelconque des revendications 1-15, dans lequel l'intervalle de glycosylation cible est un intervalle de glycosylation d'un ou plusieurs glycanes dans une préparation d'une protéine recombinante.

17. Procédé de production d'une composition pharmaceutique comprenant une protéine recombinante ayant une variabilité réduite du niveau de glycosylation entre lots, dans lequel ledit procédé comprend la réduction de la variabilité des niveaux de glycosylation entre lots de ladite protéine recombinante en effectuant un procédé selon l'une quelconque des revendications 1 à 16.

18. Procédé selon la revendication 17, comprenant en outre la combinaison de ladite protéine recombinante ayant une variabilité réduite du niveau de glycosylation entre lots avec un véhicule pharmaceutiquement acceptable, et, facultativement, la soumission de la composition pharmaceutique avec le véhicule pharmaceutiquement acceptable à la lyophilisation.

19. Procédé selon l'une quelconque des revendications 1 à 18, dans lequel ladite protéine recombinante est une protéine thérapeutique recombinante.

20. Procédé selon l'une quelconque des revendications 1 à 19, dans lequel ladite protéine recombinante est un anticorps thérapeutique recombinant.

21. Procédé selon l'une quelconque des revendications 1 à 20, dans lequel l'anticorps thérapeutique recombinant est choisi dans le groupe consistant en abatacept, abciximab, adalimumab, aflibercept, alefacept, alemtuzumab, basiliximab, belatacept, belimumab, bevacizumab, canakinumab, brentuximab vedotin, certolizumab, cetuximab, daclizumab, denileukin diftitox, denosumab, eculizumab, efalizumab, etanercept, gemtuzumab, golimumab, ibritumomab, infliximab, ipilimumab, muromonab, natalizumab, ofatumumumab, omalizumab, palivizumab, panitumumumab, ranibizumab, rilonacept, rituximab, tocilizumab, tositumomab et trastuzumab.
